# EUROPEAN PATENT SPECIFICATION

(11) **EP 3 777 935 B1**
(45) Date of publication and mention of the grant of the patent: **11.03.2026**
(21) Application number: 19191947.1
(22) Date of filing: 15.08.2019
(51) Int. Cl.: A61M 5/34, A61M 5/178, A61M 5/31

(54) **NEEDLE RETENTION DEVICE FOR RETAINING A NEEDLE OF THE NEEDLE ASSEMBLY WHEN ATTACHED TO A SYRINGE**
NADELHALTEVORRICHTUNG ZUM HALTEN EINER NADEL DER NADELEINHEIT WENN BEFESTIGT AN EINER SPRITZE
DISPOSITIF DE RETENUE D'AIGUILLE POUR RETENIR UNE AIGUILLE DE L'ENSEMBLE AIGUILLE LORSQU'IL EST FIXÉ À UNE SERINGUE

(43) Date of publication of application: 17.02.2021
(73) Proprietor: SCHOTT Pharma Schweiz AG, 9000 St. Gallen (CH)
(72) Inventor: MOSER, Raymond, 9032 Engelburg (CH)
(74) Representative: Blumbach · Zinngrebe Patentanwälte PartG mbB

(56) References cited:
- WO-A1-2010/009079
- WO-A1-2013/004675
- FR-A- 574 431
- US-A- 1 668 315
- US-A- 2 047 512

## Description

### Field of the invention

The present disclosure relates to an apparatus for injecting a fluid, comprising an injector for injecting a fluid, preferably a syringe for injecting a medical, diagnostic, cosmetic or dental preparation, for example, a needle assembly, including a needle and a hub for mounting the needle to the injector, and devices and methods to be attached to the apparatus.

### Background of the invention

Application devices such as apparatuses for injecting a fluid, comprising an injector for injecting a fluid, e.g. a syringe, are well known. Typically, such injectors may comprise a fluid that may be stored in a cavity or a container thereof. The fluid may be a liquid or semi-liquid material, for example a medical, diagnostic, cosmetic or dental preparation, to be injected into a patient's body. When said apparatus is in its operational state for injecting a fluid stored in the container, a cannula or needle is normally connected to the injector, may be coupled to a coupling portion at the distal end or mouth of the injector. For injecting the fluid stored in the container, a compression force is then applied to the fluid in order to discharge the fluid out of the container and through the inner channel of the needle.

For special applications, the fluid may be pasty or highly viscous, e.g. when injecting specific cosmetic preparations. In such a case, the compression force necessary for discharging the pasty fluid may become quickly very high. This may lead to the risk of a detachment of the needle assembly and/or the needle from the injector when injecting the fluid, if the pressure to the fluid is too high. This may be much worse when such a needle detachment takes place during an injection of the fluid.

From WO 2013/004675 A1 is known an accessory for a syringe for holding a needle on the syringe in order to avoid a risk of detachment during injection of a fluid. The needle is attached to the syringe via a so-called Luer lock connection to the distal end of the syringe, wherein a Luer lock hub of the needle assembly can be retained by the accessory. The accessory includes a bearing element that is designed and arranged such that a force exerted by the user on the accessory and which is essentially oriented parallel to the longitudinal axis of the syringe results in a force applied by the accessory on the needle which comprises a longitudinal component oriented parallel to the longitudinal axis of the syringe and a radial oriented component perpendicular to this axis and towards the center of the syringe. Thus, this accessory works together with the Luer lock portion of the needle only.

From EP 0 980 276 B1 is known a cannula holder for a syringe, wherein this cannula holder comprises a sleeve having a bore for receiving a complete syringe. The assembly of the syringe and the cannula holder is sophisticated.

From WO 2015/167150 A2 is known a syringe holder for preventing separation of the syringe needle, the syringe body comprising a syringe mounting space for accommodation a complete syringe.

From DE 10 2013 210 998 A1 is known a syringe body having an axial inner cavity, and a needle, which is connected to the syringe body, wherein the syringe body has a coupling segment at the needle-side longitudinal end of the syringe body, to which coupling segment the needle is fastened by means of a connecting element that can be slid on in the longitudinal direction. A radially inward cavity is provided on the coupling segment, which cavity can be brought into engagement or is in engagement with at least one corresponding locking segment provided on the connecting element. A special design of the syringe is required in order to form the connecting element.

From US 2010/00167829 A1 is known a further apparatus for limiting the movement of a needle assembly when coupled to a medicament container.

From US 5,925,032 A is known a syringe cannula holder, having a split outer sleeve that can be folded around the cannula. The sleeve is held in place by a sliding retaining nut, wherein a locking retainer holds the syringe within the sleeve. The assembly of the syringe and the cannula holder is complex.

From EP 1 521 608 B1 is known an accessory for a syringe for avoiding detachment of a needle from the syringe. The accessory comprises a body of semi-tubular shape for receiving the syringe. Two transverse walls are provided in order to accommodate the proximal flange or proximal lateral tabs of the body of the syringe.

From WO 2005/065753 A1 is known a cylindrical cannula hood for a syringe for securing a cannula assembly to a syringe body. The cannula hood comprises a cylindrical portion and a tapered portion, wherein the cylindrical portion is designed to fit over the syringe body and to be retained by a friction fit.

From US 5,759,178 is known a support member for stabilizing the connection of a needle and a syringe in order to avoid breakage of the nozzle of the syringe.

From EP 1 971 382 B1 is known an accessory for a needle of a syringe comprising a sleeve surrounding the hub and comprising arms for locking the sleeve in position, wherein this locking mechanism comprises latching pawls and shoulders. The safety needle accessory protects a user from needle stick injuries.

From US 1,524,242 A is known a retaining helix spring as a part of a needle retaining assembly for syringes for avoiding detachment of a needle from a syringe. The accessory comprises a syringe barrel having a discharge nipple and a helical spring resiliently engaging at its inner end the base of said nipple.

### Replacement Page 5, 5a

From US 4,490,142 A is known a syringe with a pair of clamping arms that are mounted on a ring located at the front end of a syringe frame and are biased toward closed position for preventing accidental displacement of the needle hub. The clamping mechanism is complex and requires different components, e.g. a lever member. From US 2,806,473 A, US 4,822,343 A, WO 1990/011789 A1 and EP 3 187 218 A1 similar mechanisms are known. Such syringes are often used as blood collection devices.

WO 2010/009079 Al describes a medical injector, including a medicament container, a needle assembly, a coupler, and a retainer. The retainer is configured to be coupled to the needle assembly and to an adapter that is coupled to the container. US 2 047 512 A provides hypodermic syringe container and disassemblable fittings to couple a needle to the container. FR 574 431 A describes a fin-shaped retainer that is configured to be coupled to the needle assembly. The retainer enables an operator to secure the needle assembly when injecting a fluid.

Most of the accessories and methods mentioned above are very sophisticated in their structure and therefore, costly in their production, and/or they comprise a huge number of components that have to be handled, and/or the assembly of the accessories is time-intensive when adding to a conventional syringe-needle assembly, and/or the accessories require specific and complex tools for mounting on a syringe-needle assembly.

Further problems may occur when the needle has to be replaced during or to be removed after an application process or after a medical treatment. A cosmetic treatment for example may involve multiple single injections, which may require replacing the needle by a fresh needle after some injections and during the cosmetic treatment.

Therefore, not only a safe connection of the syringe and the needle is required, that prevents the risk of needle detachment during the injection, but also the possibility to simply and quickly replace a used needle.

The replacement and/or the removal of a used needle from the injector should be a safe process for the operator, preventing the operator from being stitched by a needle, in particular preventing the operator from being stitched by a used needle. The above mentioned accessories and methods require an operator to directly touch and contact a needle during dismantling, which can be a big danger for the operator because of being stitched by the needle.

It is therefore an object of the present invention to provide an apparatus for injecting a fluid, comprising an injector for injecting a fluid, and a needle assembly, including a needle and a hub for mounting the needle to the injector, wherein said needle is attached to the injector for injecting the fluid in a safe way.

An object of the present invention is therefore to prevent the detachment of the needle from the injector when injecting a fluid, regardless of the compression force applied to the fluid and/or the viscosity of the fluid. Another object of the present invention is to provide an apparatus for injecting a fluid wherein needles of any sufficient diameter, in particular needles with a small diameter of the inner channel, can be used. Such needles typically are characterized by the diameter of the inner channel or bore, wherein the unit "Gauge" is normally used. It is an object of the invention to employ needles having a small diameter of the inner channel, e.g. in a range of a 31 Gauge needle, a 32 Gauge needle, a 33 Gauge needle or a 34 Gauge needle (a 0,260 mm needle, a 0.235 mm needle, a 0.210 mm needle or a 0.184 mm needle).

Another object of the present invention is to provide an apparatus for injecting a fluid wherein an operator can simply and quickly replace the needle.

Another object of the present invention is to provide an apparatus for injecting a fluid wherein a replacement of the needle can be performed, which prevents the operator from being stitched by the needle, in particular preventing the operator from being stitched by a used needle.

Another object of the present invention is to provide an apparatus for injecting a fluid providing a visible evidence that the needle assembly is correctly mounted onto the injector so that the apparatus is ready and safe for an application or an injection.

### Summary of the invention

The inventors have addressed the above mentioned problems and objects and have made available an apparatus for injecting a fluid, comprising an injector for injecting said fluid, preferably a syringe for injecting a medical, a diagnostic, a cosmetic or a dental preparation, a needle assembly and a needle retention device according to the independent claim.

The needle retention device according to the invention avoids the abovementioned disadvantages and therefore, affords several improvements in relation to known apparatuses for injecting a fluid with a cannula or needle. Preferred embodiments and developments of the invention are set forth in the respective subclaims.

As used in this specification, the term "injecting a fluid" generally refers to application processes, e.g. in the chemical industry, or treatment processes such as medical or cosmetic treatments, wherein a fluid is injected into a body or object, once or preferably several times, preferably with the apparatus for injecting said fluid, comprising an injector for injecting said fluid, preferably a syringe, and a needle assembly.

Therefore, the fluid may comprise liquids, gases, solids or mixtures of these. In particular, the fluid may be a liquid or semi-liquid material or a pasty or highly viscous substance.

Said needle assembly may include a needle and a hub, wherein said hub defines a contact area, preferably a shoulder.

Said injector may comprise an axial inner cavity or cylindrical container, which extends in the direction of a longitudinal axis of the injector body and in which a plunger can be or is accommodated in such a way that the plunger can be moved in the longitudinal direction. The cylindrical container is configured for receiving and containing said fluid.

The container may have a proximal end which limits the chamber at one side and into which the plunger can be inserted, and an opposite distal end, which can comprise a mouth or a conically tapering tip. The distal end may be generally of an elongate shape tapering conically towards the distal end and may have an opening in the form of a channel through which the fluid can be discharged out of the container if a suitable plunger movement takes place in a longitudinal direction.

As used in this specification, the terms "proximal" and "distal" refer to the direction closer and away from an operator when using the apparatus, respectivly. The operator can be a doctor or a nurse, for example, administering the preparation to a patient, or in any other way. It should be understood that this directional relationships are reversed when a patient administers a drug himself.

In the pharmaceutical field, such injectors are known as syringes and can be made of glass or polymer.

Said injector may further comprise a coupling portion, configured to be coupled with said hub of said needle assembly. The coupling portion may comprise said conically tapering tip and/or any other means to couple a hub of a needle assembly, for example a thread.

When said apparatus is in its operational state for injecting a fluid, said hub of said needle assembly is coupled to said coupling portion of said injector. In this manner, the needle of the needle assembly can be or is connected to the distal end of the container of the injector. When mounted, the needle of this so-called injector-needle assembly is configured to convey the fluid from the container to the patient's body through the channel or bore of the needle. Accordingly, the hub is fluid-tight connected to said coupling portion of the injector. In a preferred embodiment of the invention, the connection of the needle assembly and the injector is made such that the connection can withstand a highly pressurized fluid.

To discharge the fluid out of the container, the plunger can be moved in a longitudinal direction towards the distal end of the container. This can be done by an operator or in any other way, e.g. by or with support of any kind of mechanical devices such as a stepper motor. The movement applies the necessary compression force to the fluid stored in the container, so that the fluid can be conveyed outwards, for example to a patient's body, via the needle.

In accordance with one embodiment of the invention, the hub can be fastened by means of a connecting element that can be slid on the mouth of the injector in the longitudinal direction.

In another embodiment, the hub can be coupled to the injector by a thread, for example by a Luer lock connector. In accordance with this embodiment, the coupling portion of the injector and the hub of the needle assembly each may comprise a Luer lock connector in accordance with the international standard ISO 80369. Typically, the injector comprises a so-called female Luer lock connector, and the needle assembly comprises a so-called male Luer lock connector. The hub and the coupling portion can be or are coupled via said Luer lock connector. The coupling of the hub to the injector by a thread, preferably by a Luer lock connector, guarantees the compatibility between different manufacturers of needles, needle assemblies and injectors, and, therefore, makes it possible to use the invention for a large number of needles, needle assemblies and injectors.

The needle assembly and the injector can be coupled easily without any tools if such a Luer lock connection is available. For injecting the fluid, as mentioned above, a compression force can be applied to the fluid stored in the container in order to discharge the fluid through the distal end of the container and through the inner channel of the needle.

In accordance with the disclosure, there is provided said needle retention device, comprising a needle-retainer portion, having a finger configured to radially extend over at least a part of said contact area of said hub, and a container-contact portion, defining a first opening configured to be attached onto a peripheral section of said container, preferably in a form-fitting way.

When said hub of said needle assembly is coupled to said coupling portion of said container, said container-contact portion is attached to said container and said finger of said needle-retainer portion extends over at least a part of said contact area of said hub when said needle retention device is in its retaining position, preferably for preventing a detachment of the needle assembly from the container. In a preferred embodiment, said contact area of said hub may comprise a shoulder or any other kind of a recess, configured such that said finger of the needle-retainer portion so that the finger can snap in the shoulder or the recess.

The apparatus for injecting a fluid, comprising said needle retention device, offers an easy to handle and time-saving way for safely connecting a needle with an injector.

When adding the needle retention device to an injector-needle assembly, no tools are required.

The connection of the injector and the needle is secured by the needle retention device that prevents, when attached to the injector-needle assembly, the risk of needle detachment during the injection.

The needle retention device in accordance with the invention prevents the detachment of the needle from the injector when injecting a fluid, regardless of the compression force applied to the fluid and/or the viscosity of the fluid.

The invention allows using needles of any diameter, in particular the use of needles having a small diameter of the inner channel. Characterized by the diameter of the inner channel in the unit "Gauge", the invention makes it possible to employ very thin needles, for example small-bore needles having a size of the inner channel of less than or equal to that of a 30 Gauge needle, a 31 Gauge needle, a 32 Gauge needle, a 33 Gauge needle or a 34 Gauge needle (a 0.311 mm needle, a 0,260 mm needle, a 0.235 mm needle, a 0.210 mm needle or a 0.184 mm needle).

Thus, the invention allows the use of injectors having a volume of, for example, 0.25 ml, 0.5 ml, 0.6 ml, 1.0 ml, 1.2 ml, 1.5 ml, 2.0 ml, 2.25 ml, 3, 0 ml, 5.0 ml, 10.0 ml, 20 ml or 50 ml, together with cannulas or needles of different Gauge sizes, e.g. 27, 28, 29, 30 Gauge (0.413, 0.362, 0.337, 0.311 mm) and in particular with needles with Gauge sizes 31, 32, 33, or 34 Gauge (0.25, 0.23, 0.20, or 0.16 mm).

The needle retention device additionally offers the possibility to simply and quickly remove a used needle and/or replace a needle by another needle. Such replacements may occur during an application or treatment process. For cosmetic treatments, for example, it may be necessary to involve multiple single injections, which may require replacing a used needle after some injections by a fresh needle.

The needle retention device provides a secure replacement or removal of a needle and/or a secure assembly of a needle to the injector for an operator, preventing the operator of being stitched by a needle, in particular preventing the operator of being stitched by a used needle.

If the needle is connected to the syringe with a thread, for example with a Luer lock connection that is widespread in the pharmaceutical field, a great advantage of the invention lies in the fact that independent from the number of needle replacements, any needle is attached and secured in the same manner. This might be important since the removal of a used needle from an injector may pollute the thread by the fluid, at least to a small extent, when the fluid is leaked out of the container of the injector and/or out of the inner channel of the needle. But, if the thread is polluted with fluid, the frictional forces will be worse, e.g. the torque to loosen the connection can be significantly reduced. This may lead to a high risk for detachment of the needle during an injection.

The invention therefore allows several replacements of the needle without increasing the risk for a needle detachment since the needle is hold by the needle retention device. Therefore, the one or more needle replacements can take place even during an application or treatment process.

Thus, the invention allows the operator to save, simply and quickly remove and/or replace the needle of the injector.

The needle retention device may comprise an elongated portion, preferably extending parallel to a longitudinal axis, that may connect the needle-retainer portion and the container-contact portion to each other.

To improve the gripping of the operator, in a further embodiment the elongated portion is arranged such that it protrudes radically in order to allow an operator to hold the needle retention device in a safe way. Therefore, the elongated portion preferably does not, at least in sections, touch the outer portion of the hub or the container, but may protrude by a few millimeters from these surfaces in order to allow an operator a better handling of the needle retention device.

When the needle retention device is firmly connected to the needle assembly, the operator can easily handle the needle retention device together with the needle assembly and the needle, respectively. Accordingly, mounting and/or detaching the needle retention device and/or of the needle assembly becomes very easy and safe. The elongated portion may therefore comprise additionally any kind of a finger grip.

This makes it very helpful for the handling of used needles, because the needle, in particular a used needle, does not have to be touched by the operator. This is also very helpful when the needle is attached via a thread to the injector, since in such cases the needle has to be rotated to release the connection.

In accordance with this embodiment, the invention thus enables an easy separation and disposal of used needles. Since the needle retention device is a very cost-efficient product, the used needle can be disposed very easy together with the needle retention device, when the needle retention device is attached to the needle assembly. In order to improve further recycling activities, it could be helpful if the needle retention device is made of the same material or material group as the hub of the needle assembly.

The needle retention device is an easy to produce and cost-efficient accessory that can be made from a plasticmaterial by injecting moulding, e.g., or 3D-printing. It can be made monolithically in one-piece.

The needle retention device may comprise a thermosetting or a thermoplastic material adapted to pharmaceutical applications. Preferably, the material for the needle retention device is selected to provide a specific degree of elasticity. This allows the needle retention device to absorb higher forces in the axial direction that might occur when the fluid is very pasty or highly viscous. The yield strength that can be measured in accordance with DIN EN ISO 527-1 is preferably in the range of at least 30 N/mm² or, more preferably, in the range of at least 40 N/mm².

The needle retention device can be made of thermoplastics, including but not limiting those commonly used and approved in the medical field, accordingly including cyclo-olefin copolymer (COC), cyclo-olefin polymer (COP), acrylonitrile-butadiene-styrene (ABS), polyamide (PA), polylactate (PLA), polymethyl methacrylate (PMMA), polycarbonate (PC) or polyethylene terephthalate (PET). The yield strength of PA, for example, may be in a range of app. 50 to 80 N/mm², the yield strength of PC may be in a range of 60 N/mm² or more.

The needle retention device also can be made of thermoset plastics, including but not limiting those commonly used and approved in the medical field, accordingly including or consisting of cellulose acetate (CA) or transparent thermosetting resin.

Preferably, the material for the needle retention device is further selected to be stable to the procedures of sterilization, e.g. stable when exposed to an autoclave to methods of X-ray sterilization. Depending on the preparation, it is then also possible to sterilize a filled, in particular prefilled, injector together with the preparation, which can considerably contribute to an improved storage life, since in this case germs within the container can generally be excluded with high yield.

In just another aspect of the invention, it is therefore advantageous if the assemblies described above are X-ray or gamma-ray stable and thus accessible to X-ray or gamma-ray sterilization, which reliably precludes bacterial contamination of the prefillable or prefilled container.

In just another further advantageous embodiment of the invention, the assemblies described above are temperature stable up to 121 °C and can therefore be autoclaved.

In an embodiment of the invention, the colour of said needle retention device may be, at least partially, a signal colour, preferably different from the colour of the container and/or the needle and/or the hub. This provides a safety feature of the apparatus because the operator quickly can check whether the needle is secured in order to prevent detachment. The colour can be selected in accordance with the norm RAL in order to clearly identify safety features. For example, the needle retention device can be coloured, at least in sections, in accordance with the colour RAL 1023 ("safety yellow").

In another preferred embodiment, the needle retention device can only be attached to the injector-needle assembly if the needle assembly is completely and correct coupled to the injector. For example, this can be made such that the contact area of the hub may comprise a recess, configured such that said finger of the needle-retainer portion only can snap in when the hub is correctly mounted onto the injector. This provides an additional safety feature to the operator, because the operator can quickly check whether the needle is correctly attached to the injector. The invention therefore offers a visible evidence that the needle assembly is correctly and safely mounted to the container before the delivery of the fluid.

Thus, the invention offers a visual perception for the operator that the needle assembly is safely coupled to the coupling portion of the container and secured against detachment when the needle retention device is attached to the injector-needle assembly.

According to the invention, the needle-retainer portion comprises at least two fingers defining a second opening configured for circumferential engaging the contact area of the hub.

For this embodiment, the hub comprises a contact area in the form of a shoulder, located between a a distal end portion and a proximal end portion of the hub. The fingers may snap into said contact area, preferably said shoulder, of the hub in order to counteract an axial movement of the hub when the hub is coupled to the injector. In this manner, a detachment of the needle assembly can be prevented.

In a preferred embodiment of the invention, said second opening defines an inner diameter smaller than the outer diameter of said contact area, preferably said shoulder, such that said two fingers can snap onto said contact area, preferably said shoulder, to form a form-fitting or, more preferably, a snap fit in radial direction when mounted. This allows a firm fit of the needle retention device on the hub or the needle assembly, respectively.

Such a firm fit results in the great advantage of the invention, because for removal of the needle, or the needle assembly, respectively, the operator only has to hold on to the needle retention device. Therefore, the operator is, when removing the needle or the needle assembly, not into contact with the needle or the needle assembly. This increases the safety for the operator and prevents the operator from being stitched by the needle when removing.

In a further embodiment of the invention, said first opening of said container-contact portion completely encloses a peripheral section of container. This allows a firm fit of the needle retention device onto the injector. The needle retention device can be assembled to the injector by sliding in a longitudinal direction up to the intended position. The needle retention device can be hold in place by latching in an opposite recess, if available on the peripheral section of the container. The needle retention device can also be attached to the peripheral section of the container by adhesives.

In a further embodiment of the invention, the needle retainer portion of the needle retention device comprises more than one finger arranged parallel to each other and being spaced apart from each other, preferably defining equal distances to each other. It is preferred that the fingers are arranged such that they extend, at least in sections, in a direction parallel to the longitudinal direction of the container. This allows the fingers to encompass a peripheral section of the hub of the needle assembly in a longitudinal direction. It is further preferred that the end of the fingers comprise a radially projecting hook directed inwards to engage the contact area of the hub when mounted on the injector. In this way, several holding points for preventing axial detachment of the hub can be provided.

A very stable and evenly in axial direction holding mechanism of the hub can be achieved if the needle retainer device comprise more than two such fingers, defining equal distances to each other, and in accordance providing evenly distributed holding positions over the circumference of the contact area. In preferred embodiments, three such fingers, four fingers, five fingers or six fingers or even more are provided.

In just another embodiment of the invention, the needle retention device may further comprise a locking ring, defining an opening configured for circumferential enclosing, at least in sections, the container-contact portion of the needle retention device. For this embodiment, it is preferred that the needle retainer device comprises at least two such fingers arranged parallel to each other and being spaced apart from each other, preferably defining equal distances to each other and, preferably, arranged such that they extend, at least in sections, in a direction parallel to the longitudinal direction of the container.

In this embodiment, said locking ring is configured for rotating around the axis of said container when mounted, having recesses for receiving said fingers when in a first, opened position when said fingers do not extend over the contact area of the hub, preferably the shoulder of the hub, and pressing said fingers onto said shoulder when in a second, closed position. Mechanically acting stops or interengaging latch means may be provided to form an end stop at the first and second rotational positions.

In just another embodiment of the invention, the needle retention device further comprises a sliding ring, defining an opening configured for circumferential enclosing, at least in sections, the container-contact portion of the needle retention device. Also, for this embodiment, it is preferred that the needle retainer device comprise at least two such fingers arranged parallel to each other and being spaced apart from each other, preferably defining equal distances to each other and, preferably, arranged such that they extend, at least in sections, in a direction parallel to the longitudinal direction of the container.

In this embodiment, said sliding ring is configured for axial movement along the axis of said container when mounted, releasing said fingers when in a first axial position, and pressing said fingers into said contact area of the hub, preferably the shoulder of the hub, when in a second axial position.

The invention provides in a further embodiment an apparatus for injecting a fluid, comprising an injector for injecting said fluid, preferably a syringe for injecting a medical, a diagnostic, a cosmetic or a dental preparation, a needle assembly and a needle retention device, wherein the container of the apparatus is filled, completely or at least to a small amount, with a fluid. The fluid may be for example a medical, a diagnostic, a cosmetic or a dental preparation.

The fluid may comprise preparations suitable for use in the hyaluronic acid segment for cosmetics, medial of opthalmics applications, for example.

The fluid may comprise preparations suitable for use in the veterinary segment for anti-parasite applications, for example.

The fluid may comprise preparations suitable for use in the infusion therapy segment for infusion therapy applications, for example.

The fluid may comprise preparations suitable for use in the neuroleptics segment for schizophrenia medication, for example.

The fluid may comprise preparations suitable for use in the biologics segment for biotech, sensitive molecules or sensitive protein applications, for example.

The fluid may comprise preparations suitable for use in other segments too, for diluent or cancer treatment, for emergency drugs or for vaccines, for example.

In a further example of the disclosure, there is provided a needle retention device for an apparatus for injecting a fluid, comprising an injector for injecting a fluid, preferably a syringe for injecting a medical, diagnostic, cosmetic or dental preparation preparation, comprising a needle-retainer portion, having a finger configured to radially extend over at least a part of a contact area of a hub of a needle assembly when coupled to a container of an injector of the apparatus, and a container-contact portion, defining a first opening configured to be attached in a form-fitting way onto a peripheral section of said container of said injector.

When the needle assembly is coupled to a coupling portion of the injector, the container-contact portion is attached to the container and the finger of the needle-retainer portion extends over at least a part of the contact area of the hub when the needle retention device is in its retaining position, preferably for preventing a detachment of the needle assembly from the container.

The disclosure provides in a further example the use of an apparatus for injecting a fluid, comprising an injector for injecting a fluid, preferably a syringe for injecting a medical, diagnostic, cosmetic or dental preparation preparation.

When using the apparatus for injecting the fluid, the fluid may have a viscosity in a range of at least 1 mPa*s, more preferably of at least 10 mPa*s or 12 mPa*s, or 15 mPa*s.

When using the apparatus for injecting the fluid, the needle of the apparatus may comprise small-bore needles having a size of the inner channel of less than or equal to that of a 30 Gauge needle, a 31 Gauge needle, a 32 Gauge needle, a 33 Gauge needle or a 34 Gauge needle.

When using the apparatus for injecting the fluid, preferably the needle retention device prevents an detachment of said needle assembly from the injector when injecting the fluid, wherein the maximum force that can be applied to the fluid is at least 50 N, preferably at least 70 N and more preferably at least 90 N 100 N.

The disclosure thus also provides a method comprising injecting or applying a fluid from or with an apparatus in accordance with the invention. The fluid may comprise liquids, gases, solids or mixtures of these. In particular, the fluid may be a liquid or semi-liquid material or a pasty or highly viscous substance.

For injecting the fluid, a needle assembly including a needle and a hub may be coupled to an injector, wherein the container of the injector is configured for receiving and containing said fluid.

Specific injections require the fluid being plasty or having a high viscosity. For example, when injecting cosmetic preparations like Botulinum toxin, also called botulinum or botulinum neurotoxin, for beauty treatments, such preparations may have a viscosity in a range of at least 1 mPa*s.

In a similar way, applications such as medical treatments may comprise Hyaluronic acid preparations that are injected into arthrosis-damaged joints of a body in order to lubricate the joint and act as a "shock absorber", for example. When injecting such hyaluron preparations, the viscosity of the preparation may also be in a range of at least 1 mPa*s.

Also fluids comprising a preparation in other segments as mentioned above may have such a similar viscosity.

In order to enable injections of such highly viscous fluids, the connection of the needle and the container is secured by the needle retention device of the invention such that the connection can withstand a highly pressurized fluid in the container. Thus, the needle retention device of the invention prevents, when attached to an injector-needle assembly, the detachment of the needle or needle assembly, respectively, from the injector when injecting such fluids.

For injecting the fluid, a compression force is to be applied to the fluid stored in the container in order to discharge the fluid through the distal end of the container and through the inner channel of the needle. The compression force can be applied to the fluid by a movement of a plunger in a longitudinal direction from the proximal end to the distal end of the container. This can be done by an operator, for example.The movement of the plunger applies the necessary compression force to the fluid stored in the container, so that the fluid can be conveyed outwards, for example to a patient's body, via the needle.

The invention enables injecting a fluid having a viscosity in a range of at least 1 mPa*s, more preferably of at least 10 mPa*s, or 12 mPa*s, or 15 mPa*s. The viscosity may be measured with a viscometer in accordance with the norm EN ISO 3219.

Especially when such highly viscous liquids are used, the pressure to the fluid when moving the plunger may become relatively high very quickly. This danger is exacerbated when the needle assembly comprises very thin needles, for example small-bore needles having a size of the inner channel of less than or equal to that of a 30 Gauge needle.

The invention enables injecting a fluid from or with an apparatus in accordance with the invention, wherein the injector-needle assembly may be exposed to a pressurized fluid having a pressure of almost 5 bar, 10 bar, 15 bar or even 20 bar or more. Even under these conditions, the needle is not being detached from the injector.

The maximum force that can be applied to the fluid stored in the container of the apparatus in accordance with the invention when injecting the fluid is up to at least 50 N, preferably up to at least 70 N and more preferably up to at least 90 N or 100 N, without detachment of said needle assembly from the injector.

A force of about 100 N is above the force of app. 95 N for a male and app. 64 N for a female normally reached when a male or a female operator, respectively, is using an apparatus in accordance with the invention, e.g. a syringe, and is applying a force to the finger pad of the plunger. Therefore, the apparatus in accordance with the invention safely resists the maximum forces that an operator manually can apply to the apparatus when pressing his finger against the finger pad in order to apply the compression force to the fluid.

At the same time, the invention enables injecting a fluid from or with an apparatus in accordance with the invention, wherein the needles of the needle assembly comprise small-bore needles having a size of the inner channel of less than or equal to that of a 30 Gauge needle, a 31 Gauge needle, a 32 Gauge needle, a 33 Gauge needle or a 34 Gauge needle needle (a 0.311 mm needle, a 0,260 mm needle, a 0.235 mm needle, a 0.210 mm needle or a 0.184 mm needle).

The disclosure thus also provides a method comprising injecting a fluid from or with an apparatus according to the invention, comprising a "ready-to-use" prefilled container, via a needle with a Gauge size equal to that of a 30 Gauge needle, a 31 Gauge needle, a 32 Gauge needle, a 33 Gauge needle or a 34 Gauge needle (a 0.311 mm needle, a 0,260 mm needle, a 0.235 mm needle, a 0.210 mm needle or a 0.184 mm needle).

The disclosure thus also provides a cosmetic method comprising injecting a cosmetic preparation using an apparatus according to the invention, wherein the container is prefilled with a liquid, preferably with a cosmetic preparation, and in which the needle is having a Gauge size equal to that of a 30 Gauge needle, a 31 Gauge needle, a 32 Gauge needle, a 33 Gauge needle or a 34 Gauge needle (a 0.311 mm needle, a 0,260 mm needle, a 0.235 mm needle, a 0.210 mm needle or a 0.184 mm needle).

Further details of the invention will become apparent from the description of the illustrated embodiments and the appended claims.

### Brief description of the drawings

The above-mentioned and other features and advantages of this invention, and the manner of attaining them, will become more transparent and the invention will be better understood by reference to the following description of exemplary embodiments of the invention taken in conjunction with the accompanying drawings, wherein:
- Fig. 1: is a perspective view of a detail of an apparatus for injecting a fluid, comprising an injector for injecting a fluid, a needle assembly and a needle retention device, according to a first embodiment of the invention,
- Fig. 2: is a perspective view of a detail of an apparatus for injecting a fluid, comprising an injector for injecting a fluid, a cap for closing the mouth of the injector, and a needle retention device, according to a second embodiment of the invention,
- Fig. 3: is a perspective view of a detail of an apparatus for injecting a fluid, comprising an injector for injecting a fluid and a needle retention device having several elongated fingers, wherein the injector comprises a Luer lock connector, according to a third embodiment of the invention,
- Fig. 4: is a perspective view of a detail of an apparatus for injecting a fluid, comprising an injector for injecting a fluid and a needle retention device having several elongated fingers and a sliding ring, according to a fourth embodiment of the invention,
- Fig. 5: is a perspective view of a detail of an apparatus for injecting a fluid, comprising an injector for injecting a fluid, wherein the injector comprises a Luer lock connector, a needle assembly and a needle retention device having several elongated fingers and a sliding ring, according to a fifth embodiment of the invention,
- Fig. 6: is a perspective view of a detail of an apparatus for injecting a fluid, comprising an injector for injecting a fluid, wherein the injector comprises a Luer lock connector, a needle assembly and a needle retention device having several elongated fingers and a sliding ring, in accordance with the embodiment of Fig. 4,
- Figs. 7a, 7b, 7c: are perspective views of a detail of an apparatus for injecting a fluid, comprising an injector for injecting a fluid, wherein the injector comprises a Luer lock connector, and a needle retention device having several elongated fingers and a sliding ring, in accordance with the embodiment of Fig. 4, showing different positions of the sliding ring,
- Figs. 8a, 8b, 8c, 8d, 8e, 8f: are different views of a detail of an apparatus for injecting a fluid, comprising an injector for injecting a fluid, wherein the injector comprises a Luer lock connector, and a needle retention device having several elongated fingers and a locking ring, according to a sixth embodiment of the invention, showing different positions of the locking ring,
- Figs. 9a, 9b, 9c, 9d: are perspective views of a detail of an apparatus for injecting a fluid, comprising an injector for injecting a fluid, wherein the injector comprises a Luer lock connector, and a needle retention device having three elongated fingers and a sliding ring, according to a seventh embodiment of the invention, showing different positions of the sliding ring,
- Fig. 10: is a diagram showing extrusion forces during tests,
- Fig. 11: is a perspective view of a detail of an apparatus for injecting a fluid, comprising an injector for injecting a fluid, a needle and a needle retention device, according to the embodiment of Fig. 2,
- Figs. 12a, 12b, 12c, 12d: are different views of a detail of an apparatus, showing the functionality of the locking ring,
- Fig. 13: is just another view of the front side of locking ring,
- Fig. 14: is a plan view of two caps and two needle assemblies,
- Figs. 16a, 16b and 16c: are plan views of differents types of needle assemblies sufficient for use with the invention,
- Fig. 17: is a perspective view of an embodiment of a needle retention device,
- Fig. 18: is a perspective view of a detail of an apparatus for injecting a fluid, comprising an injector for injecting a fluid, a hub of a needle assembly, and a needle retention device,
- Fig. 19: is just another perspective view of a detail of an apparatus for injecting a fluid, comprising an injector for injecting a fluid, a hub of a needle assembly, and a needle retention device, and
- Fig. 20: is a draft of the sliding mechanism of the sliding ring.

Corresponding reference characters indicate corresponding parts throughout the several views. The exemplifications set out herein illustrate embodiments of the invention and such exemplifications are not construed as limiting the scope of the invention in any manner.

### Detailed description of the drawings

The invention provides an apparatus for injecting a fluid, comprising an injector for injecting said fluid, preferably a syringe for injecting a medical, a diagnostic, a cosmetic or a dental preparation, a needle assembly and a needle retention device.

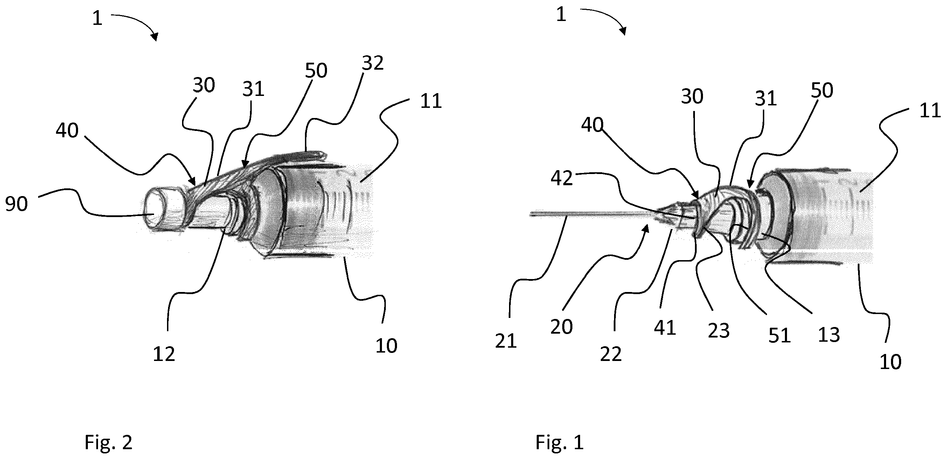

Referring now to the drawings, and more particulary to Fig. 1, there is shown a first embodiment of such an apparatus 1 for injecting a fluid, comprising an injector 10 for injecting said fluid, preferably a syringe for injecting a medical, a diagnostic, a cosmetic or a dental preparation, a needle assembly 20 and a needle retention device 30. The fluid is a liquid or semi-liquid material or a pasty or highly viscous substance.

Fig. 2 is a perspective view of a detail of an apparatus 1 for injecting a fluid, comprising an injector 10 for injecting a fluid, a cap 90 for closing the mouth of the injector 10, and a needle retention device 30, according to a second embodiment of the invention.

The needle assembly 20 as shown in Fig. 1 includes a needle 21 and a hub 22, wherein said hub 22 defines a contact area 23, preferably a shoulder. In this comparative example, the contact area 23 is construed as a shoulder.

The injector 10 comprises a container 11, which extends in the direction of a longitudinal axis of the injector body and in which a plunger can be accommodated in such a way that the plunger can be moved in the longitudinal direction. The cylindrical container 11 is configured for receiving and containing said fluid. In the example of the first embodiment of the invention, the injector 10 is a syringe as typically used in the pharmaceutical field, having a container volume of 10 ml. The syringe is made of a polymer material. Such syringes can be supplied from the company SCHOTT AG, Mainz, under the name "TopPac SD", for example, in a huge variety of sizes.

The injector 10 further comprises a coupling portion, configured to be coupled with the hub 22 of the needle assembly 20.

When the apparatus 1 is in its operational state for injecting a fluid as shown in the embodiment of Fig. 1, the hub 22 of said needle assembly 20 is coupled to the coupling portion 12 of the injector 10. This injector-needle assembly of Fig. 1 is therefore ready for an injection.

Regarding the apparatus 1 of Fig. 1, the connection of the needle 21 and the container 11 is made such that the connection can withstand a highly pressurized fluid in the container 11. To discharge the fluid out of the container 11, a plunger can be moved in a longitudinal direction towards the distal end of the container. This can be done by an operator or in any other way, e.g. by a mechanical device like a stepper motor. Such a movement applies the compression force to the fluid stored in the container, so that the fluid can be conveyed outwards, for example to a patient's body, via the needle 21.

In accordance with the embodiment of Fig. 1, the hub 22 is fastened by means of a connecting element to slid on the mouth of the injector 10 in a longitudinal direction.

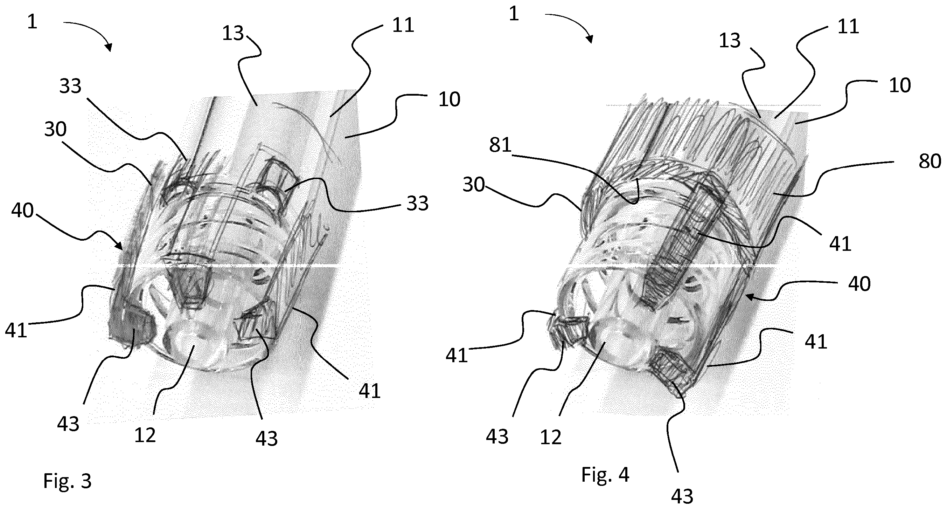

In another embodiment of the invention, the hub 22 can be coupled to the injector 10 by a thread, for example by a Luer lock connector. An injector 10, also suitable for the invention and comprising such a Luer lock connection, can be seen in Figs 3 and 4, for example.

Fig. 3 is a perspective view of a detail of an apparatus 1 for injecting a fluid, comprising an injector 10 for injecting a fluid and a needle retention device 30 having several elongated fingers 41, wherein the injector 10 comprises a Luer lock connector, according to a third embodiment of the invention. The embodiment of the needle retention device 30 Fig. 3 comprises embedded bumps 33 on the container for rotational opening the fingers 41.

Fig. 4 is a perspective view of a detail of an apparatus 1 for injecting a fluid, comprising an injector 10 for injecting a fluid and a needle retention device 30 having several elongated fingers 41 and a sliding ring 80, according to a fourth embodiment of the invention. The opening of the struts or fingers 41 is performed by rotation and/or axial movement of the sliding ring 80.

As shown in the embodiments of Figs. 3 and 4, the coupling portion 12 of the injector 10 and the hub 22 of the needle assembly 20 each comprise a Luer lock connector in accordance with the international standard ISO 80369. The hub 22 and the coupling portion 12 are coupled via said Luer lock connector.

The coupling of the hub 22 to the injector 10 by such a Luer lock connector guarantees the compatibility between the different manufacturers of needles 21, needle assemblies 20 and injectors 10, and, therefore, makes it possible to use the invention for a large number of needles 21, needle assemblies 20 and injectors 10.

In accordance with one aspect of the invention and as shown in Fig. 1, there is provided a needle retention device 30, comprising a needle-retainer portion 40, having a finger 41 configured to radially extend over at least a part of said contact area 23 of said hub 22, and a container-contact portion 50, defining a first opening 51 configured to be attached onto a peripheral section 13 of said container 11, preferably in a form-fitting way. The needle-retainer portion 40 and the container-contact portion 50 are connected to each other by an elongated portion 31, arranged in a longitudinal direction.

As also shown in Fig. 1, said hub 22 of said needle assembly 20 is coupled to said coupling portion 12 of the injector 10 representing a injector-needle assembly. The needle retention device 30 is attached to this injector-needle assembly in its retaining position, wherein the container-contact portion 50 is attached to said container 11 and the finger 41 extends over at least a part of the contact area of said hub 22. In this manner, the needle retention device 30 prevents a detachment of the needle assembly 20 from the injector 10.

The apparatus 1 for injecting a fluid in accordance with the invention offers an easy to handle and time-saving way for safely connecting a needle 21 with an injector 10 by providing said needle retention device 30.

When adding the needle retention device 30 to an injector-needle assembly, no tools are required.

The connection of the injector 10 and the needle 21 is secured by the needle retention device 20 that prevents, when attached to the injector-needle assembly, the risk of needle detachment during an injection or an application or treatment process.

The needle retention device 30 in accordance with the invention prevents the detachment of the needle 21 from the injector 10 when injecting a fluid, up to very high compression forces applied to the fluid and/or the viscosity of the fluid.

It could be shown in comparative tests that a force of app. 105 N, corresponding to a pressure of the fluid in the container of app. 33 bar, can be applied to a 1 ml glass syringe that is filled with the fluid, wherein the syringe is combined with a standard needle from the supplier "Terumo", without a needle pop off. Only leakages have been observed.

In further tests, it could be shown that syringes made of polymer, e.g. "TopPac HVD" from SCHOTT AG, have better characteristics in terms of needle pop off and leakage than needles made of glass.

In additional tests it could be shown for apparatuses, comprising such syringes made of a polymer material, that the mean value for leakage and needle pop off is between 1.6 and 2.3 times better when using the needle retention device of the invention.

The invention allows using needles 21 of any diameter, in particular the use of needles 21 having a small diameter of the inner channel, making it possible to employ very thin needles 21, for example small-bore needles 21 having a size of the inner channel of less than or equal to that of a 30 Gauge needle, a 31 Gauge needle, a 32 Gauge needle, a 33 Gauge needle or a 34 Gauge needle (a 0.311 mm needle, a 0,260 mm needle, a 0.235 mm needle, a 0.210 mm needle or a 0.184 mm needle).

Thus the invention allows the use of injectors 10 having a typical volume of, for example, 0.25 ml, 0.5 ml, 0.6 ml, 1.0 ml, 1.2 ml, 1.5 ml, 2.0 ml, 2.25 ml, 3, 0 ml, 5.0 ml, 10.0 ml, 20 ml or 50 ml, together with cannulas or needles 21 of different Gauge sizes, e.g. 27, 28, 29, 30 Gauge (0.413, 0.362, 0.337, 0.311 mm) and in particular with needles with Gauge sizes 31, 32, 33, or 34 Gauge (0.25, 0.23, 0.20, or 0.16 mm).

The needle retention device 30 additionally offers the possibility to simply and quickly remove a used needle 21 and/or replace a needle 21 by another needle 21. Such replacements may occur during an application or treatment process. For cosmetic treatments, for example, it may be necessary to involve multiple single injections, which may require replacing a used needle 21 after some injections by a fresh needle 21.

The needle retention device 30 provides a secure replacement or removal of a needle 21 and/or a secure assembly of a needle 21 to the injector 10 for an operator, preventing the operator of being stitched by a needle 21, in particular preventing the operator of being stitched by a used needle 21.

The invention is very advantageous for injector-needle assemblies, when the needle 21 is connected to the injector 10 with a thread, for example with a Luer-Lock connection as shown in Figs. 3 and 4. In such cases, the removal of a used needle from an injector may pollute the thread by the fluid, at least to a small extent, when the fluid is leaked out of the container 11 of the injector 10 and/or out of the inner channel of the needle 21. If the thread is polluted with fluid, the frictional connection will be worse, e.g. the torque to loosen the connection can be significantly reduced. This may lead to a high risk for detachment of the needle during injection.

Therefore, the invention offers the great advantage because the detachment of the needle 21 from the injector 10 is prevented independent from the torque to loosen the connection. Thus, any needle 21 is attached and secured in the same manner to the injector 10.

The invention allows the operator to save, simply and quickly remove and/or replace the needle 21 of the apparatus 1 at the end of use. For a good grip for an operator, in a further embodiment the needle retention device 30 comprises an elongated portion 31, which protrudes radically in order to allow an operator to hold the needle retention device 30 in a safe way.

Therefore, the elongated portion 31 as shown in Fig. 1 does not, at least in sections, touch the outer portion of the hub 22, but protrudes at least app. 5 millimeters from the surfaces in order to allow an operator a better handling of the needle retention device 30. In general, it is preferred when the elongated portion 31 protrudes from the outer surface of the hub 22 and/or the container 11 at least 3 millimeters, preferred at least 4 millimeters and more preferred of least 5 millimeters, at least 7 millimeters, at least 10 millimeters. On the other hand, the handling of the complete apparatus 1 should not be adversely affected by such a protrusion, therefore the protrusion should be less than app. 50 mm, preferred less than 40 mm and more preferred less than 30 mm.

In a further embodiment of the invention, the needle retention device 30 comprises an additional extended elongated portion 32 as shown in the embodiment of Fig. 2. The additional extended elongated portion 32 is made such that an operator can hold the needle retention device 30 securely. Of course, additional finger pads can be foreseen to improve the operator when gripping the needle retention device 30.

The great advantage is that for the handling of used needles 21, the needle 21, in particular a used needle 21, does not have to be touched directly by the operator. This is very helpful when the needle 21 is attached via a thread to the injector 10 as shown in the embodiments of Fig. 3 and 4, for example, since in such cases the needle 21 and/or the needle assembly 20 has to be rotated compared to the container to release the connection. It is helpful if the elongated portion 31 and/or the extended elongated portion 32 does not protrude more than 30 mm for a better handling when rotating.

Thus, the invention enables an easy separation and disposal of used needles 21 or needle assemblies 20, respectively.

Since the needle retention device 30 is a very cost-efficient product, the used needle 21 can be disposed very easy together with the needle retention device 30, when the needle retention device is attached to the needle assembly.

The needle retention device 30 is an easy to produce and cost-efficient accessory that can be made from a plastic material by injecting moulding, e.g., or 3D-printing. The needle retention device 30 is made monolithically in one-piece from cyclo-olefin copolymer (COC).

The needle retention device 30 can be made of other thermoplastics, including but not limiting those commonly used and approved in the medical field, accordingly including cyclo-olefin polymer (COP), acrylonitrile-butadiene-styrene (ABS), polyamide (PA), Polylactate (PLA), polymethyl methacrylate (PMMA), polycarbonate (PC) or polyethylene terephthalate (PET).

The needle retention device 30 also can be made of thermoset plastics, including but not limiting those commonly used and approved in the medical field, accordingly including or consisting of cellulose acetate (CA) or transparent thermosetting resin.

The material for the needle retention device 30 is selected to provide a specific degree of elasticity. This allows the needle retention device to absorb higher forces in the axial direction that might occur when the fluid is very pasty or highly viscous.

The material for the needle retention device 30 is stable to the procedures of sterilization, i.e. the needle retention device 30 is X-ray and/or gamma-ray stable and thus accessible to X-ray or gamma-ray sterilization, which reliably precludes bacterial contamination of the prefillable or prefilled container.

The material for the needle retention device 30 is also temperature stable up to 121 °C and can therefore be autoclaved.

The needle retention device 30 is coloured in "safety yellow" (RAL 1023) in order to provide an additional safety feature of the apparatus 1 because the operator quickly can check whether the needle 21 is secured for preventing detachment.

In a preferred embodiment of the invention, the needle-retainer portion 40 comprises two fingers 41 defining a second opening configured for circumferential engaging the contact area of the hub 22.

Such embodiment is shown in Fig. 1, wherein the hub 22 comprises a contact area 23 in the form of a shoulder, located between a distal end portion and a proximal end portion of the hub 22. The fingers 41 are disposed against said shoulder of the hub 22 in order to counteract an axial movement of the hub 22 when the hub 22 is coupled to the injector 10. In this manner, a detachment of the needle assembly 20 from the injector 10 can be prevented.

In the embodiments shown in Figs. 3 and 4, the needle retainer portion 40 of the needle retention device 30 comprises more than one finger 41 arranged parallel to each other and being spaced apart from each other, preferably defining equal distances to each other. It is preferred that the fingers 41 are arranged such that they extend, at least in sections, in a direction parallel to the longitudinal direction of the container 11. This allows the fingers to encompass an peripheral section of the hub 22 of the needle assembly 20 in a longitudinal direction.

The end of the fingers 41 further comprise a radially projecting hook 43 directed inwards to engage the contact area 23 of the hub 22 when mounted on the injector 10. In this way, several holding points for preventing axial detachment of the hub 22 can be provided.

A very stable and evenly in axial direction holding mechanism of the hub 22 can be achieved if the needle retention device 30 comprise more than two such fingers 41, defining equal distances to each other, and in accordance providing evenly distributed holding positions over the circumference of the contact area. In the embodiments as shown in Fig. 4, e.g., three such fingers 41 are provided, defining three holding position distributed over the circumference of the contact area 23 of the hub 22.

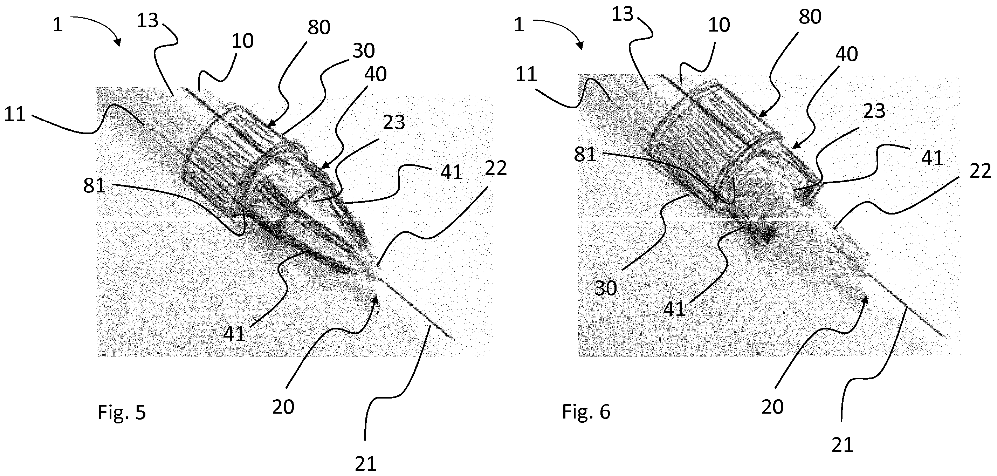

In Fig. 5 is shown a perspective view of a detail of an apparatus 1 for injecting a fluid, comprising an injector 10 for injecting a fluid, wherein the injector 10 comprises a Luer lock connector, a needle assembly 20 and a needle retention device 30 having several elongated fingers 41 and a sliding ring 80, according to a fifth embodiment of the invention.

In Fig. 6 is shown a perspective view of a detail of a similar apparatus 1 for injecting a fluid, also comprising a Luer lock connector, a needle assembly 20 and a needle retention device 30 having several elongated fingers 41 and a sliding ring 80, in accordance with the embodiment of Fig. 4.

The needle retention device 30 of Fig. 6 comprises two fingers 41, wherein the needle retention device 30 in accordance with Fig. 5 comprises four such fingers 41.

The sliding ring 80 is configured for axial movement along the axis of the container 11 when mounted, releasing said fingers 41 when in a first axial position, and pressing the fingers 41 into the contact area 23 of the hub 22, preferably the shoulder of the hub 22, when in a second axial position. Figs. 5 and 6 show the sliding ring 80 in the second axial position for locking the hub 22 by pressing the fingers 41 into the contact area 23.

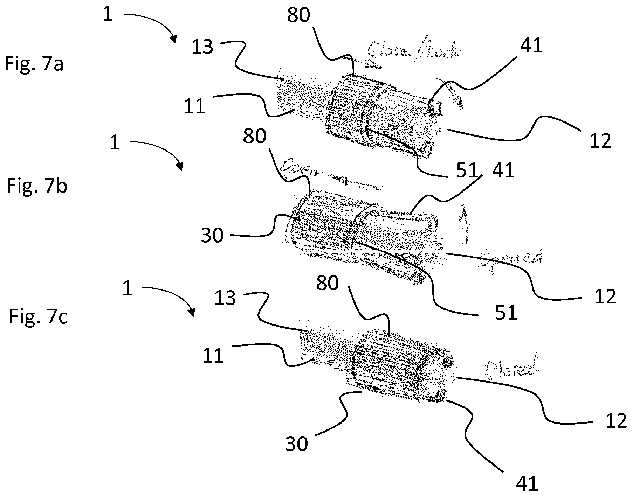

In Figs. 7a, 7b and 7c are shown perspective views of a detail of an apparatus 1 for injecting a fluid in accordance with the embodiment of Fig. 4. The figures show different positions of the sliding ring 80.

The needle retention device 30 comprises a sliding ring 80, defining an opening 81, also called opening sliding ring 81, configured for circumferential enclosing, at least in sections, the container 11.

The opening 81 of the sliding ring 80 is preferably dimensioned such that it can completely enclose the container 11 with only a little radial tolerance, but enough to allow the necessary axial movement.

Also, for this embodiment, it is preferred that the needle retention device 30 comprise at least two such fingers 41 as shown in the figures 7a, 7b and 7c, arranged parallel to each other and being spaced apart from each other, preferably defining equal distances to each other and, preferably, arranged such that they extend, at least in sections, in a direction parallel to the longitudinal direction of the container.

Fig. 7a shows a neutral position of the sliding ring 80, from which the sliding ring 80 can be axially moved in a proximal direction as shown in Fig. 7b or in a opposite direction to the distal end of the injector 10 as shown in Fig. 7c.

When in the proximal direction, corresponding to the first axial position, the sliding ring 80 enables the fingers 41 to open and to unlock the hub 22. As shown in Fig. 7b, the hook 43 of the fingers 41 can be removed radially outwards in this position of the sliding ring 80 for enabling a loosening of the hub 22.

As shown in Fig. 7c, the sliding ring 80 is in the second axial position, equivalent to a closed position, and thereby locking the hub 22 by pressing the fingers 41 into the contact area 23.

As shown in this embodiment, said sliding ring 80 is configured for axial movement along the axis of said container 11 when mounted, releasing said fingers 41 when in a first axial position or open position, and pressing said fingers 41 into said contact area of the hub 22, preferably the shoulder of the hub 22, when in a second axial position or closed position.

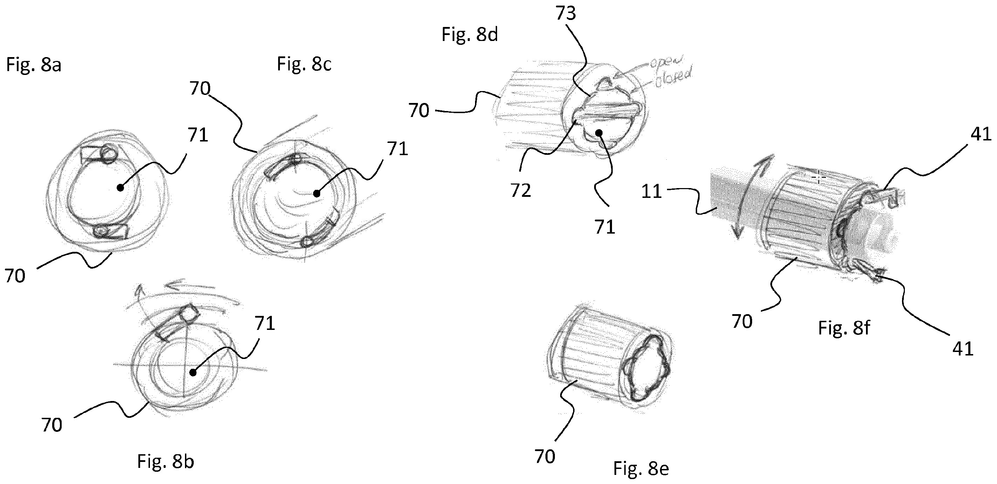

Figures 8a, 8b, 8c, 8d, 8e and 8f show different views of a detail of an apparatus 1 for injecting a fluid, comprising an injector 10 for injecting a fluid, wherein the injector 10 comprises a Luer lock connector, and a needle retention device having 30 several elongated fingers 41 and a locking ring 70, according to a sixth embodiment of the invention, showing different positions of the locking ring 70. The locking ring 70 defines an opening 71, also called opening locking ring 71, configured for circumferential enclosing, at least in sections, the container 11.

As best can be seen in Fig. 8f, the locking ring 70 interacts with the fingers 41 such that the locking ring 70 is configured for rotating around the axis of said container 11 when mounted, having recesses 72 in the inner wall of the opening 71 for receiving said fingers 41 when in a first, opened position when said fingers 41 do not extend over the contact area of the hub 22, preferably the shoulder of the hub 22, and pressing said fingers 41 onto said contact area of the hub 22, preferably the shoulder when in a second, closed position. Preferably, the recesses 72 extend in an axial direction and dimensioned such that they can, at least partially, include at least portions of the fingers 41.

Mechanically acting stops or interengaging latch means may be provided to form an end stop at the first and second rotational positions of the locking ring 70.

As best can be seen in Fig. 8d, the inner wall of the opening 71 comprises recesses 72 and protrusions 73. The number and the position of the recesses 72 corresponds to the number and the position of the fingers 41 in order to enable unlocking the fingers 41 when the locking ring 70 is in the opened position. Also, the number and the position of the protrusions 73 correspondes to the number and the position of the fingers 41 in order to press the fingers 41 onto the contact area of the hub 22, preferably the shoulder, when the locking ring 70 in the closed position.

Figs. 8a-8c show the locking ring 70 in a front view, illustrating different arangements of the protrusions 73 and the recesses 72.

For this embodiment, it is also preferred that the needle retention device 30 comprise at least two such fingers 41 arranged parallel to each other and being spaced apart from each other, preferably defining equal distances to each other and, preferably, arranged such that they extend, at least in sections, in a direction parallel to the longitudinal direction of the container 11.

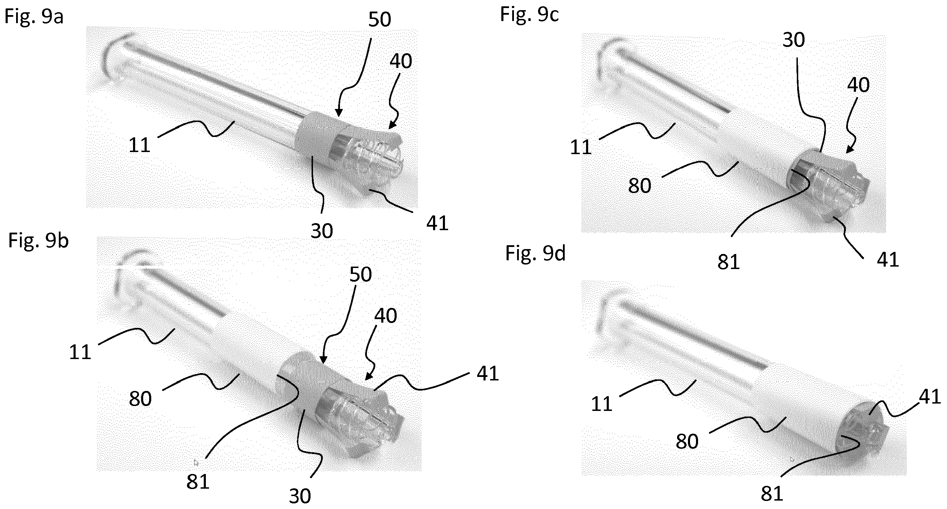

Figs. 9a, 9b, 9c and 9d show perspective views of a detail of an apparatus 1 for injecting a fluid, similar to the embodiment of Fig. 4. The needle retention device 30 comprises three elongated fingers 41 and a sliding ring 80. In Fig. 9a the needle retention device 30 is shown without the sliding ring 80. Figs. 9b, 9c and 9d illustrate the sliding ring 80 in different positions.

As shown in Fig. 9a, the needle retention device 30 comprises a container-contact portion 50, having a first opening that encloses a peripheral section 13 of the container 11. In this manner, it can be, for example, easiliy attached to the container 11 by axial sliding onto the container 10. Further protrusions and/or recesses can be provided in order to form a firm connection between the needle retention device 30 and the container 11 with form-fitting. This connection can be secured, for example, by the sliding ring 80, when the sliding ring 80 is in the second axial position for locking the fingers 41. Fig. 9d shows the sliding ring 80 in this second axial position for locking the fingers 41.

Alternatively or additionally, the sliding ring 80 can press the container-contact portion 50 of the needle retention device 30 to the container 11 in order to establish a friction fit between the needle retention device 30 and the container 11 when the the sliding ring 80 is in the second axial position for locking the fingers 41. The inner surface of the first opening 51 of the container-contact portion 50 may comprise, at least in sections, a rough portion to increase the friction.

In addition, adhesives may be used to fix the needle retention device 30 and the container 11. But, these adhesives have to be selected such that they are suitable for use in the pharmaceutical field. Also, the use of adhesives is not sufficient for handling the needle with the needle retention device 30, for example after usage of the needle.

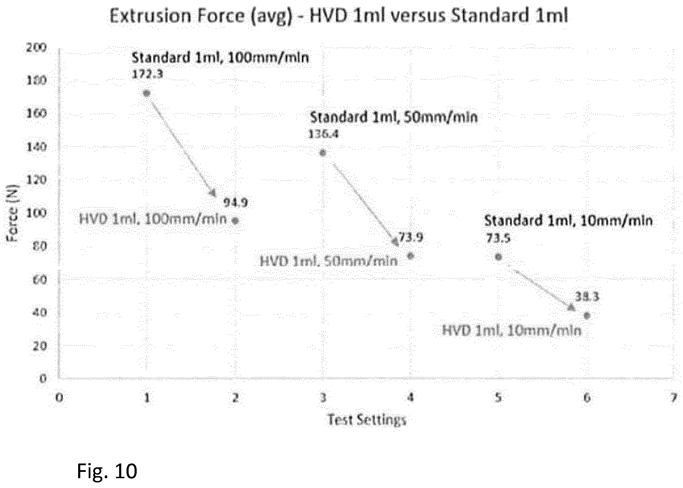

Fig. 10 illustrates the extrusion force measured during tests with high viscous fluids. A small needle of 31 G was used. Very high applied forces have been observed which could lead to a needle pop off if the assembly of the needle and the syringe is not safe. A low assembly torque and/or contamination of the thread and/or the use of small needles may aggravate this problem. When no needle retention device 30 is used, there is no visible evidence that the needle assembly is safe before the application of the fluid, in particular under high forces.

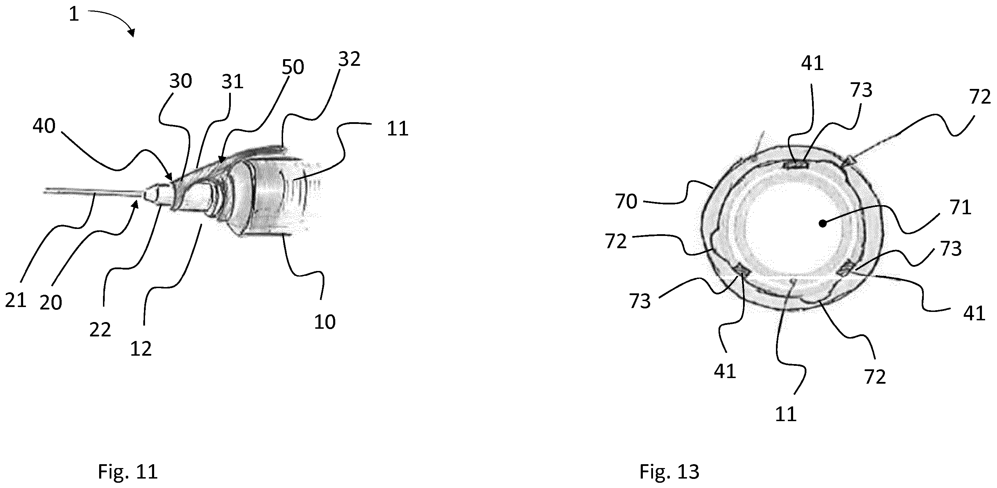

Fig. 11 shows a perspective view of a detail of an apparatus 1 for injecting a fluid, comprising an injector 10 for injecting a fluid, a needle assembly 20 and a needle retention device 30, according to the embodiment of Fig. 2.

The needle retention device 30 of this embodiment can be made very costly since since it can be produced as a single piece accessory. The needle retention device 30 comprises a extended elongated portion 32 that is constructed as a grip for easy handling. The grip enables a handling or a detachment of the needle assembly 20 without touching the needle 21, for example after usage of the needle.

The needle-retainer portion 40 comprises two fingers 41, allowing a fixation around the hub 22. The container-contact portion additionally comprises a first opening 51 to be engaged with a groove on the injector 10.

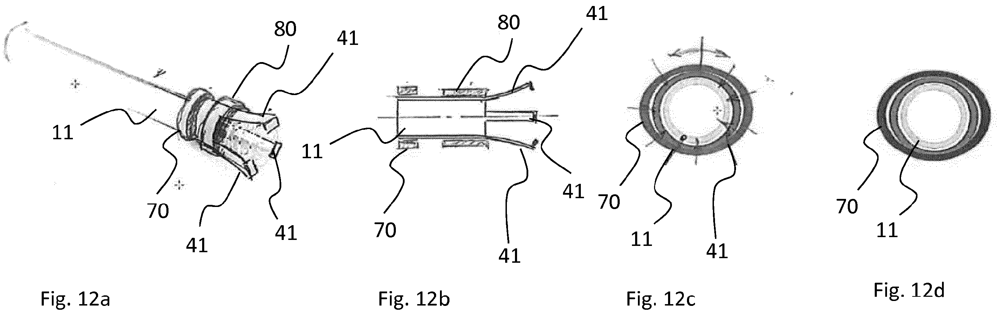

Figs. 12a, 12b, 12c, 12d are different views of a detail of an apparatus 1, showing the functionality of the locking ring 70 and the sliding ring 80. An objective of the invention is to avoid any modifications of the container 11 of the injector 10. In order to avoid these modifications, the invention provides also a squeezing mechanism. In addition or alternatively, the fingers 41 can be glued to the container 11. But, as mentioned above, using adhesives causes some disadvantages that have to be taken into account.

The general principle of the locking ring 70 and the sliding ring 80 is shown in the Figs. 12a, 12b, 12c and 12d, wherein the locking ring 70 is used to lock the assembly together, and the sliding ring 80 is used to close and open the fingers 41 by sliding motion and rotation to lock the fingers 41 in opened and closed positions.

Fig. 13 shows just another view of the front side of locking ring 70. In this embodiment, the locking ring 70 is arranged such that it can lock three fingers 41.

For locking and unlocking the three fingers 41, three recesses 72 for unlocking and three protrusions 73 for locking the fingers are provided at the inner surface of the opening 71. The opening and closing of the fingers 41 can be achieved through rotation of the locking ring 70 as the legs enter the recesses 72 within the locking ring 70.

In an additional embodiment of the invention, the locking ring 70 and/or the sliding ring 80 and/or the needle retention device can be made, at least in sections, of a transparent material. This allows to see the locking status of the apparatus 1.

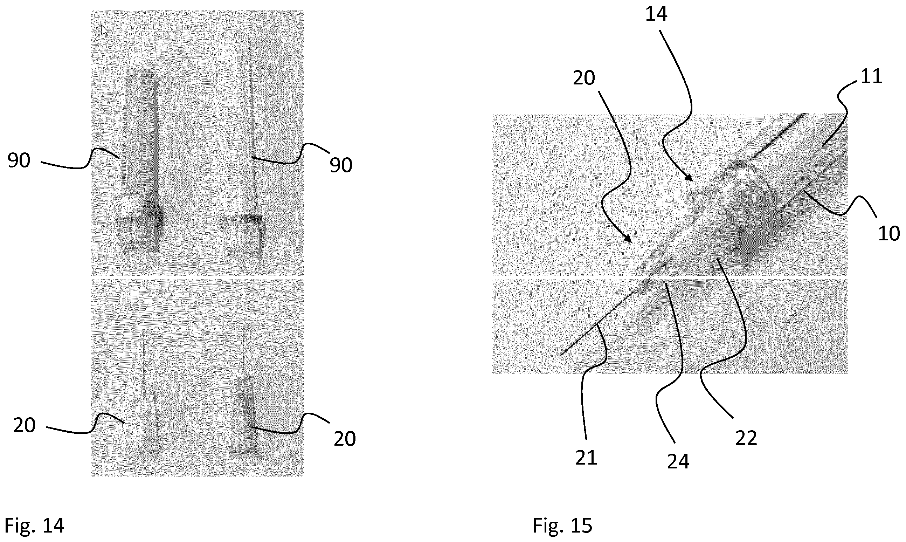

Fig. 14 show in a plan view two different types of caps 90 and two different types of needle assemblies 20. Normally, the caps 90 can be used for secure handling when mounting the needle assembly 20 to the injector 10. Before application of a fluid, the cap 90 can be removed and the needle retention device 30 can be assembled to the injector 10.

Fig. 15 illustrates a needle assembly 20 mounted to a syringe. The connection is made by a Luer lock connection, comprising a Luer lock connector 14. The hub 22 of the needle assembly is made with notches, defining a shoulder. This shoulder enables the fingers 41 to snap in or to engage with when in a locking position and, therefore, prevents the detachment of the needle assembly 20 from the injector 10, in particular when injecting a fluid.

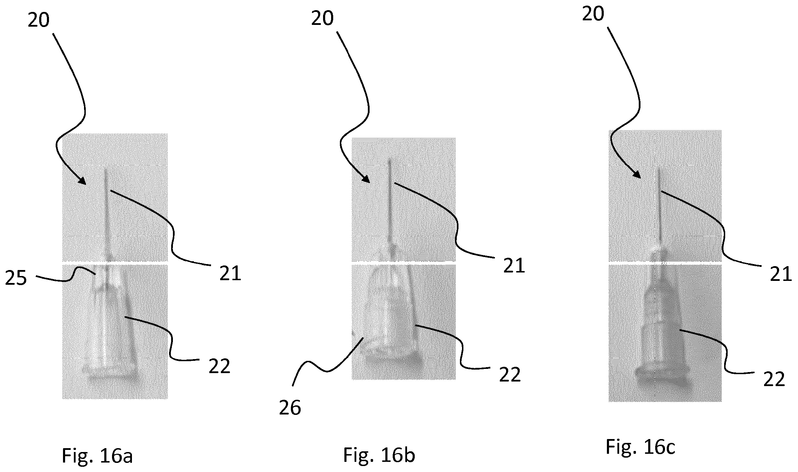

Figs. 16a, 16b and 16c show plan views of differents types of needle assemblies 20 sufficient for use with the invention. Fig. 16a shows a needle assembly 20 having a needle 21 of the size 30 G x ½ Zoll (0.311 mm x 12.7 mm) that can be bought from the company Merz Pharma. This needle assembly 20 comprises fixation fins 25.

Fig. 16b shows a needle assembly 20 having a 30 G x ½ Zoll (0.311 mm x 12.7 mm) needle 21 and a Luer lock connector. Fig. 16c shows just another needle assembly 20 that can be bought from the company Terumo.

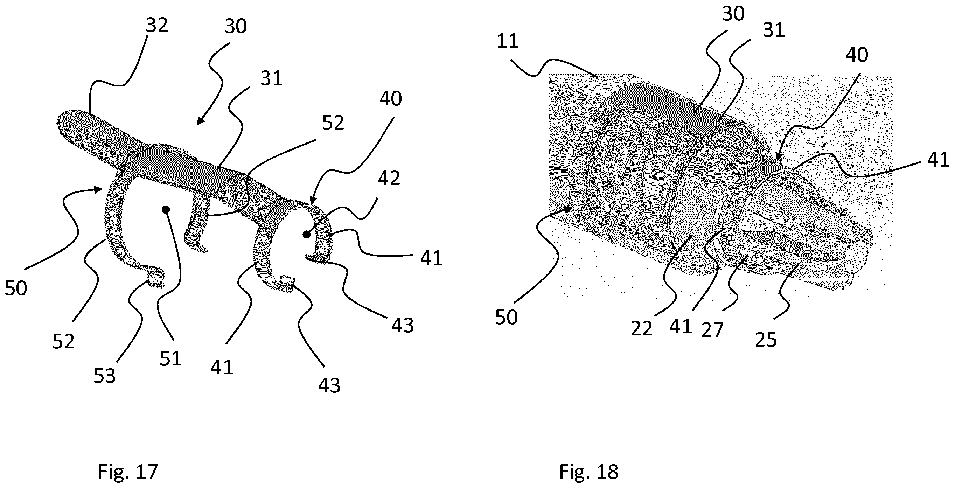

Fig. 17 shows a perspective view of an embodiment of a needle retention device 30, comprising two fingers 41, each including a hook 43 to engage with a hub 22 of a needle assembly 20. The container-contact portion 50 of this embodiment of the needle retention device 30 also comprises two contact fingers 52, defining the first opening 51, and arranged to encompass a peripheral section 13 of an injector 10. The end portions 53 of the contact fingers 52 are widened to allow an easy and quick assembly onto the container 11.

Fig. 18 shows a perspective view of a detail of an apparatus 1 for injecting a fluid, comprising an injector 10 for injecting a fluid, a hub 22 of a needle assembly 20, and a needle retention device 30.

In this embodiment, the hub 22 comprises fins 25 defining a shoulder 27 that allows a hook 43 of a finger 41 to snap in for establishing a form fit connection.

It can be understood and seen from the figures that such an assembly of a needle assembly 20 and an injector 10 is safe since the needle assembly 20 can be screwed within the luer lock. The needle assembly 20 can removed by screwing out the needle assembly 20. The needle retention device 30 enables an operator to hold the hub 22 without stiching himself with the needle 21.

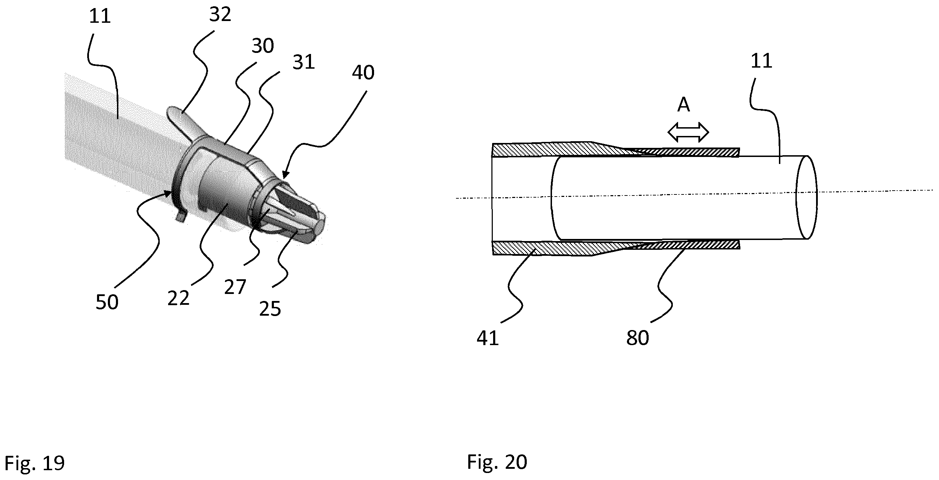

Fig. 19 shows just another perspective view of a detail of an apparatus 1 for injecting a fluid, comprising an injector 10 for injecting a fluid, a hub 22 of a needle assembly 20, and a needle retention device 30.

It can be seen from Fig. 19 that the fingers 41 are placed within the notches or shoulder 27 of the hub 22. A rotation of the needle retention device 30 and of the fingers 41 in an axial movement will unscrew the hub 22 out of Luer lock connector, and the needle 21 (not shown in this view) can be disposed in a boxe for used needles, for example, without any close contact of the needle 21 with the hand of the operator. Thus, this decreases the risk of being stiched for an operator when detaching a (used) needle. After disposing a used needle, for example, a new needle could be easily mounted within the luer lock, at the same time the needle 21 is protected within the cap 90 during the screwing of the needle 21 as shown in Fig. 14.

Finally, Fig. 20 shows a draft of the sliding mechanism of the sliding ring 80. The sliding ring 80 is designed for axial movement in two opposite axial directions as indicated by the arrow A.

### List of reference signs:

- 1: Apparatus
- 10: Injector
- 11: Container
- 12: Coupling Portion
- 13: Peripheral Section
- 14: Luer Lock Connector (female)

- 20: Needle Assembly
- 21: Needle
- 22: Hub
- 23: Contact Area
- 24: Notch
- 25: Fins
- 26: Luer Lock Connector (male)
- 27: Shoulder

- 30: Needle Retention Device
- 31: Elongated Portion
- 32: Extended Elongated Portion
- 33: Bumps

- 40: Needle-Retainer Portion
- 41: Finger
- 42: Second Opening
- 43: Hook

- 50: Container-Contact Portion
- 51: First Opening
- 52: Contact Finger
- 53: End Portion

- 70: Locking Ring
- 71: Opening locking ring
- 72: Recess
- 73: Protrusion

- 80: Sliding Ring
- 81: Opening sliding ring

- 90: Cap

## Claims

1. Apparatus (1) for injecting a fluid, comprising
an injector (10) for injecting the fluid,
preferably a syringe for injecting a medical, diagnostic, cosmetic or dental preparation,
a needle assembly (20) and
a needle retention device (30),
said needle assembly (20) including
a needle (21) and
a hub (22),
wherein said hub (22) defines a contact area (23),
said injector (10) comprising
a cylindrical container (11) configured for receiving and containing said fluid, preferably said medical preparation,
a coupling portion (12) configured to be coupled with said hub (22) of said needle assembly (20),
said needle retention device (30) comprising
a needle-retainer portion (40)
comprising at least two fingers (41)
configured to radially extend over at least a part of said contact area (23) of said hub (22), and defining a second opening (42) configured for circumferential engaging a shoulder (23) of said hub (22),
a container-contact portion (50),
defining a first opening (51) configured to be attached in a form-fitting way onto a peripheral section (13) of said container (11),
wherein
when said apparatus (1) is in its operational state for injecting a fluid,
said hub (22) of said needle assembly (20) is coupled to said coupling portion (12) of said container (11),
said container-contact portion (50) is attached to said container (11) and
each of said fingers (41) of said needle-retainer portion (40) extends over at least a part of said contact area (23) of said hub (22) when said needle retention device (30) is in its retaining position, for preventing a detachment of the needle assembly (20) from the container (11).

2. The apparatus (1) as claimed in claim 1, wherein said needle retention device (30) comprises a thermosetting or a thermoplastic material adapted to pharmaceutical applications.

3. The apparatus (1) as claimed in claim 1 or 2, wherein the colour of said needle retention device (30) is, at least partially, a signal colour, preferably different from the colour of the container or the needle or the hub.

4. The apparatus (1) as claimed in claim 1, 2 or 3, wherein said second opening (42) defines an inner diameter smaller than the outer diameter of said shoulder (23) such that said at least two fingers (41) snap onto said shoulder (23) to form a snap fit in radial direction when mounted.

5. The apparatus (1) as claimed in any of the preceding claims, wherein said first opening (51) of said container-contact portion (50) completely encloses said peripheral section (13) of said container (11).

6. The apparatus (1) as claimed in any of the preceding claims, wherein said coupling portion (12) and said hub (22) each comprise a Luer lock connector, and wherein said hub (22) and said coupling portion (12) are connected via said Luer lock.

7. The apparatus (1) as claimed in any of the preceding claims, wherein said needle retention device (30) further comprises an additional elongated portion (31), preferably extending parallel to a longitudinal axis of said container (11), defining a finger grip, for detaching said needle retention device (30).

8. The apparatus (1) as claimed in any of the preceding claims, wherein said needle retainer portion (40) comprises the fingers (41) arranged parallel to each other and being spaced apart from each other, preferably defining equal distances to each other.

9. The apparatus (1) as claimed in any of the preceding claims, wherein said needle retention device (30) further comprises a locking ring (70), defining an opening (71) configured for circumferential enclosing said container-coupling portion (50), wherein preferably said locking ring (70) is configured for rotating around the axis of said container (11) when mounted, having recesses (72) for receiving said fingers (41) when in a first, opened position when said fingers (41) do not extend over said shoulder (23), and pressing said fingers (41) onto said shoulder (23) when in a second, closed position.

10. The apparatus (1) as claimed in any of the preceding claims 1 to 8, wherein said needle retention device (30) further comprises a sliding ring (80), defining an opening (81) configured for circumferential enclosing said container-coupling portion (50), wherein preferably said sliding ring (80) is configured for axial movement along the axis of said container (11) when mounted, releasing said fingers (41) when in a first axial position, and pressing said fingers (41) into said shoulder (23) when in a second axial position.

11. The apparatus (1) as claimed in any of the preceding claims 1 to 10, wherein said needle (21) comprise small-bore needles having a size of the inner channel of less than or equal to that of a 30 Gauge needle, a 31 Gauge needle, a 32 Gauge needle, a 33 Gauge needle or a 34 Gauge needle (a 0.311 mm needle, a 0,260 mm needle, a 0.235 mm needle, a 0.210 mm needle or a 0.184 mm needle).

12. The apparatus (1) as claimed in any of the preceding claims, wherein said container (11) of said apparatus (1) is completely or at least to a small amount, filled with a fluid, preferably with a medical, a diagnostic, a cosmetic or a dental preparation.

13. The apparatus (1) as claimed in the preceding claim, wherein the fluid has a viscosity in a range of at least 1 mPa*s, more preferably of at least 10 mPa*s or 12 mPa*s, or 15 mPa*s.

14. The apparatus (1) as claimed in any of the preceding claims 11, 12 or 13, wherein said needle retention device (30) prevents an detachment of said needle assembly (20) from the injector (10) when injecting the fluid, wherein the maximum force that can be applied to the fluid is at least 50 N, preferably at least 70 N and more preferably at least 100 N.

15. The needle retention device (30) attached to the apparatus (1) according to one of preceding claims 1 to 14.

## Patentansprüche

1. Vorrichtung (1) zum Injizieren einer Flüssigkeit, umfassend:
einen Injektor (10) zum Injizieren der Flüssigkeit, vorzugsweise eine Spritze zum Injizieren eines medizinischen, diagnostischen, kosmetischen oder zahnmedizinischen Präparats,
eine Nadeleinheit (20) und
eine Nadelhaltevorrichtung (30),
wobei die Nadeleinheit (20) eine Kanüle (21) und einen Nadelansatz (22) umfasst,
wobei der Nadelansatz (22) einen Kontaktbereich (23) bestimmt,
wobei der Injektor (10) Folgendes umfasst:
einen zylindrischen Behälter (11), der zum Aufnehmen und Halten der Flüssigkeit, vorzugsweise des medizinischen Präparats, ausgebildet ist,
einen Verbindungsabschnitt (12), der dazu ausgebildet ist, mit dem Nadelansatz (22) der Nadeleinheit (20) verbunden zu werden,
wobei die Nadelhaltevorrichtung (30) Folgendes umfasst:
einen Nadelrückhalteabschnitt (40),
der mindestens zwei Finger (41) umfasst, die derart ausgebildet sind, dass sie sich radial über zumindest einen Teil des Kontaktbereichs (23) des Nadelansatzes (22) erstrecken, und
der eine zweite Öffnung (42) bestimmt, die derart ausgebildet ist, dass sie in Umfangsrichtung an einer Schulter (23) des Nadelansatzes (22) anliegt,
einen Behälterkontaktabschnitt (50),
der eine erste Öffnung (51) bestimmt, die dazu ausgebildet ist, formschlüssig an einem Randbereich (13) des Behälters (11) befestigt zu werden,
wobei, wenn sich die Vorrichtung (1) in ihrem Funktionszustand zum Injizieren einer Flüssigkeit befindet,
der Nadelansatz (22) der Nadeleinheit (20) mit dem Verbindungsabschnitt (12) des Behälters (11) verbunden ist,
der Behälterkontaktabschnitt (50) an dem Behälter (11) befestigt ist und
die Finger (41) des Nadelrückhalteabschnitts (40) sich jeweils über zumindest einen Teil des Kontaktbereichs (23) des Nadelansatzes (22) erstrecken, wenn sich die Nadelhaltevorrichtung (30) in ihrer Rückhaltestellung befindet, um eine Loslösung der Nadeleinheit (20) von dem Behälter (11) zu verhindern.

2. Vorrichtung (1) nach Anspruch 1, wobei die Nadelhaltevorrichtung (30) ein duroplastisches oder ein thermoplastisches Material umfasst, das für pharmazeutische Anwendungen geeignet ist.

3. Vorrichtung (1) nach Anspruch 1 oder 2, wobei die Farbe der Nadelhaltevorrichtung (30) zumindest zum Teil eine Signalfarbe ist, die vorzugsweise verschieden von der Farbe des Behälters oder der Kanüle oder des Nadelansatzes ist.

4. Vorrichtung (1) nach Anspruch 1, 2 oder 3, wobei die zweite Öffnung (42) einen Innendurchmesser bestimmt, der kleiner ist als der Außendurchmesser der Schulter (23), so dass die mindestens zwei Finger (41) auf die Schulter (23) schnappen, um im befestigten Zustand eine Schnapppassung in radialer Richtung zu bilden.

5. Vorrichtung (1) nach einem der vorstehenden Ansprüche, wobei die erste Öffnung (51) des Behälterkontaktabschnitts (50) den Randbereich (13) des Behälters (11) vollständig umschließt.

6. Vorrichtung (1) nach einem der vorstehenden Ansprüche, wobei der Verbindungsabschnitt (12) und der Nadelansatz (22) jeweils einen Luer-Lock-Verbinder umfassen, und wobei der Nadelansatz (22) und der Verbindungsabschnitt (12) über die Luer-Lock-Verbindung verbunden werden.

7. Vorrichtung (1) nach einem der vorstehenden Ansprüche, wobei die Nadelhaltevorrichtung (30) ferner einen zusätzlichen länglichen Abschnitt (31) umfasst, der sich vorzugsweise parallel zu einer Längsachse des Behälters (11) erstreckt, der einen Fingergriff zum Abnehmen der Nadelhaltevorrichtung (30) bildet.

8. Vorrichtung (1) nach einem der vorstehenden Ansprüche, wobei der Nadelrückhalteabschnitt (40) die Finger (41) umfasst, die parallel zueinander angeordnet und voneinander beabstandet sind, vorzugsweise in gleichen Abständen zueinander.

9. Vorrichtung (1) nach einem der vorstehenden Ansprüche, wobei die Nadelhaltevorrichtung (30) ferner einen Verriegelungsring (70) umfasst, der eine Öffnung (71) bildet, die dazu ausgebildet ist, den Behälterkontaktabschnitt (50) in Umfangsrichtung zu umschließen, wobei der Verriegelungsring (70) vorzugsweise dazu ausgebildet ist, bei der Befestigung um die Achse des Behälters (11) gedreht zu werden, wobei er Ausnehmungen (72) aufweist, zum Aufnehmen der Finger (41) in einer ersten, geöffneten Stellung, wenn die Finger (41) sich nicht über die Schulter (23) erstrecken, und wobei er in einer zweiten, geschlossenen Stellung die Finger (41) auf die Schulter (23) drückt.

10. Vorrichtung (1) nach einem der vorstehenden Ansprüche 1 bis 8, wobei die Nadelhaltevorrichtung (30) ferner einen Gleitring (80) umfasst, der eine Öffnung (81) bildet, die dazu ausgebildet ist, den Behälterkontaktabschnitt (50) in Umfangsrichtung zu umschließen, wobei der Gleitring (80) vorzugsweise zur axialen Verschiebung entlang der Achse des Behälters (11) bei der Befestigung ausgebildet ist, wobei in einer ersten axialen Stellung die Finger (41) freigeben sind, und in einer zweiten axialen Stellung die Finger (41) auf die Schulter (23) drücken.

11. Vorrichtung (1) nach einem der vorstehenden Ansprüche 1 bis 10, wobei die Nadel (21) Kanülen mit kleinem Lumen umfasst, deren Innenkanal eine Größe kleiner oder gleich der einer 30-Gauge-Kanüle, einer 31-Gauge-Kanüle, einer 32-Gauge-Kanüle, einer 33-Gauge-Kanüle oder einer 34-Gauge-Kanüle aufweist (einer 0,311 mm-Kanüle, einer 0,260 mm-Kanüle, einer 0,235 mm-Kanüle, einer 0,210 mm-Kanüle oder einer 0,184 mm-Kanüle).

12. Vorrichtung (1) nach einem der vorstehenden Ansprüche, wobei der Behälter (11) der Vorrichtung (1) vollständig oder zumindest zu einem kleinen Teil mit einer Flüssigkeit gefüllt ist, vorzugsweise mit einem medizinischen, einem diagnostischen, einem kosmetischen oder einem zahnmedizinischen Präparat.

13. Vorrichtung (1) gemäß dem vorstehenden Anspruch, wobei die Flüssigkeit eine Viskosität in einem Bereich von mindestens 1 mPa·s aufweist, vorzugsweise von mindestens 10 mPa·s oder 12 mPa·s oder 15 mPa·s.

14. Vorrichtung (1) nach einem der vorstehenden Ansprüche 11, 12 oder 13, wobei die Nadelhaltevorrichtung (30) eine Loslösung der Nadeleinheit (20) von dem Injektor (10) verhindert, wenn die Flüssigkeit injiziert wird, wobei die maximale Kraft, die auf die Flüssigkeit ausgeübt werden kann, mindestens 50 N beträgt, vorzugsweise mindestens 70 N und weiter bevorzugt mindestens 100 N.

15. Nadelhaltevorrichtung (30), die an der Vorrichtung (1) nach einem der vorstehenden Ansprüche 1 bis 14 angebracht ist.

## Revendications

1. Dispositif (1) pour injecter un fluide, comprenant un injecteur (10) pour injecter le fluide, de préférence une seringue pour injecter une préparation médicale, diagnostique, cosmétique ou dentaire,
un ensemble d'aiguille (20) et
un dispositif de retenue d'aiguille (30),
ledit ensemble d'aiguille (20) incluant
une aiguille (21) et
un embout (22),
dans lequel ledit embout (22) définit une surface de contact (23),
ledit injecteur (10) comprenant
un contenant cylindrique (11) configuré pour recevoir et contenir ledit fluide, de préférence ladite préparation médicale,
une partie de couplage (12) configurée pour être couplée audit embout (22) dudit ensemble d'aiguille (20),
ledit dispositif de retenue d'aiguille (30) comprenant
une partie de retenue d'aiguille (40) comprenant au moins deux doigts (41) configurés pour s'étendre radialement au-dessus d'au moins une partie de ladite surface de contact (23) dudit embout (22), et définissant une seconde ouverture (42) configurée pour venir en prise circonférentiellement avec un épaulement (23) dudit embout (22),
une partie de contact de contenant (50), définissant une première ouverture (51) configurée pour être fixée par ajustement de forme sur une section périphérique (13) dudit contenant (11),
dans lequel quand ledit dispositif (1) est dans un état opérationnel pour injecter un fluide, ledit embout (22) dudit ensemble d'aiguille (20) est couplé à ladite partie de couplage (12) dudit contenant (11), ladite partie de contact de contenant (50) est fixée sur ledit contenant (11) et chacun desdits doigts (41) de ladite partie de retenue d'aiguille (40) s'étend au-dessus d'au moins une partie de ladite surface de contact (23) dudit embout (22) quand ledit dispositif de retenue d'aiguille (30) est dans sa position de retenue, pour empêcher un détachement dudit ensemble d'aiguille (20) du contenant (11).

2. Dispositif (1) selon la revendication 1, dans lequel ledit dispositif de retenue d'aiguille (30) comprend un matériau thermodurcissable ou thermoplastique adapté aux applications pharmaceutiques.

3. Dispositif (1) selon la revendication 1 ou 2, dans lequel la couleur dudit dispositif de retenue d'aiguille (30) est, au moins en partie, une couleur signalétique, de préférence différente de la couleur du contenant ou de l'aiguille ou de l'embout.

4. Dispositif (1) selon la revendication 1, 2 ou 3, dans lequel ladite seconde ouverture (42) définit un diamètre intérieur inférieur au diamètre extérieur dudit épaulement (23) de telle manière que lesdits au moins deux doigts (41) s'emboîtent sur ledit épaulement (23) pour former un ajustement par emboîtement dans une direction radiale une fois montés.

5. Dispositif (1) selon l'une quelconque des revendications précédentes, dans lequel ladite première ouverture (51) de ladite partie de contact de contenant (50) enferme complètement ladite section périphérique (13) dudit contenant (11).

6. Dispositif (1) selon l'une quelconque des revendications précédentes, dans lequel ladite partie de couplage (12) et ledit embout (22) comprennent chacun un raccord luer-lock, et dans lequel ledit embout (22) et ladite partie de couplage (12) sont connectés via ledit raccord luer-lock.

7. Dispositif (1) selon l'une quelconque des revendications précédentes, dans lequel ledit dispositif de retenue d'aiguille (30) comprend en outre une partie allongée supplémentaire (31), s'étendant de préférence parallèle à un axe longitudinal dudit contenant (11), définissant une prise pour doigts, pour détacher ledit dispositif de retenue d'aiguille (30).

8. Dispositif (1) selon l'une quelconque des revendications précédentes, dans lequel ladite partie de retenue d'aiguille (40) comprend les doigts (41) agencés parallèles les uns aux autres et étant espacés les uns des autres, de préférence définissant des distances égales entre eux.

9. Dispositif (1) selon l'une quelconque des revendications précédentes, dans lequel ledit dispositif de retenue d'aiguille (30) comprend en outre une bague de verrouillage (70), définissant une ouverture (71) configurée pour enfermer circonférentiellement ladite partie de contact de contenant (50), dans lequel ladite bague de verrouillage (70) est configurée pour tourner autour de l'axe dudit contenant (11) quand elle est montée, comportant des évidements (72) pour recevoir lesdits doigts (41) quand elle est dans une première position ouverte quand lesdits doigts (41) ne s'étendent pas au-dessus dudit épaulement (23), et presser lesdits doigts (41) sur ledit épaulement (23) quand elle est dans une seconde position fermée.

10. Dispositif (1) selon l'une quelconque des revendications précédentes 1 à 8, dans lequel ledit dispositif de retenue d'aiguille (30) comprend en outre une bague coulissante (80), définissant une ouverture (81) configurée pour enfermer circonférentiellement ladite partie de contact de contenant (50), dans lequel de préférence ladite bague coulissante (80) est configurée pour un mouvement axial le long de l'axe dudit contenant (11) quand elle est montée, libérant lesdits doigts (41) quand elle est dans une première position axiale, et pressant lesdits doigts (41) dans ledit épaulement (23) quand elle est dans une seconde position axiale.

11. Dispositif (1) selon l'une quelconque des revendications précédentes 1 à 10, dans lequel ladite aiguille (21) comprend des aiguilles à petit alésage ayant une taille du canal intérieur inférieure ou égale à celle d'une aiguille de calibre 30, une aiguille de calibre 31, une aiguille de calibre 32, une aiguille de calibre 33 ou une aiguille de calibre 34 (une aiguille de 0,311 mm, une aiguille de 0,260 mm, une aiguille de 0,235 mm, une aiguille de 0,210 mm ou une aiguille de 0,184 mm).

12. Dispositif (1) selon l'une quelconque des revendications précédentes, dans lequel ledit contenant (11) dudit dispositif (1) est complètement ou au moins à une petite quantité, rempli d'un fluide, de préférence d'une préparation médicale, diagnostique, cosmétique ou dentaire.

13. Dispositif (1) selon l'une quelconque des revendications précédentes, dans lequel le fluide a une viscosité dans une plage d'au moins 1 mPa*s, plus préférablement d'au moins 10 mPa*s, ou 12 mPa*s, ou 15 mPa*s,

14. Dispositif (1) selon l'une quelconque des revendications précédentes 11, 12 ou 13, dans lequel ledit dispositif de retenue d'aiguille (30) empêche un détachement dudit ensemble d'aiguille (20) de l'injecteur (10) lors de l'injection du fluide, dans lequel la force maximum qui peut être appliquée au fluide est au moins de 50 N, de préférence au moins de 70 N et plus préférablement au moins de 100 N.

15. Dispositif de retenue d'aiguille (30) fixé sur le dispositif (1) selon l'une quelconque des revendications précédentes 1 à 14.
